Europäisches Patentamt

European Patent Office

Office européen des brevets

(11) Publication number: **0 291 686 B1**

## EUROPEAN PATENT SPECIFICATION

(45) Date of publication of patent specification: **13.10.93**

(51) Int. Cl.5: **A61K 37/64**, A61K 37/02, C12N 15/00

(21) Application number: **88105781.4**

(22) Date of filing: **12.04.88**

(54) Inhibitors of angiogenin.

(30) Priority: **14.04.87 US 38008**
**05.04.88 US 177942**

(43) Date of publication of application:
**23.11.88 Bulletin 88/47**

(45) Publication of the grant of the patent:
**13.10.93 Bulletin 93/41**

(84) Designated Contracting States:
**AT BE CH DE ES FR GB GR IT LI LU NL SE**

(56) References cited:

**PROC. NATL. ACAD. SCI., USA vol. 84, Apr. 1987, pages 2238-2241 R. SHAPIRO et al.:"Human placental ribonuclease inhibitor abolishes both angiogenic and ribonucleolytic activities of angiogenin"**

**BACR/CRC/ICRF Symposium in growth factors, London, Dec. 1987 & Br. J. CANCER, vol. 57, no. 6, 1988, pages 587-590 J.F. RIORDAN et al.:"Human angiogenin, an organogenic protein"**

(73) Proprietor: **THE PRESIDENT AND FELLOWS OF HARVARD COLLEGE**
**17 Ouincy Street**
**Cambridge, MA 02138(US)**

(72) Inventor: **Shapiro, Robert**
**181 Highland Street**
**Holliston, MA 01746(US)**
Inventor: **Vallee, Bert L.**
**56 Brown Street**
**Brookline, MA 02146(US)**

(74) Representative: **Bizley, Richard Edward et al**
**HEPWORTH LAWRENCE BRYER & BIZLEY**
**2nd Floor**
**Gate House South**
**West Gate**
**Harlow, Essex CM20 1JN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

BIOCHEMISTRY, vol. 25, 1986, pages 3527-3532 R. SHAPIRO et al.: "Characteristics ribonucleolytic activity of human an-giogenin"

INT.J. PEPTIDE PROTEIN RES., vol. 19, 1982, pages 372-379 L.F. BURTON et al.: "Ribonuclease inhibitors from the livers of five mammalian species"

IMPORTANT ADV. ONCOL, 1985, pages 42-62, GB J. FOLKMAN: "Angiogenesis and its in-hibitors"

BIOCHEMISTRY, vol. 27, 1988, pages 8545-8553 F.S. LEE et al.: "Primary structure of human placental ribonuclease inhibitor"

**Description**

Angiogenin is a human protein which induces blood vessel formation (Fett et al., 24 Biochemistry 5480, 1985). This biological activity is expressed with an amount as low as 35 fmol (using a chick embryo CAM assay procedure, Id.). Although originally isolated from medium conditioned by human tumor cells, angiogenin is not tumor specific, can be found in a variety of other cells and biological fluids, and most likely plays a role in normal and/or pathological neovascularization. It has a molecular weight of about 14,400 and an isoelectric point greater than about pH9.5, Id. Strydom et al. 24 Biochemistry 5486, 1985 also disclose the amino acid sequence of angiogenin.

Angiogenin is known to have an enzymatic activity. Specifically, it catalyzes limited cleavage of 28S and 18S rRNA to produce a specific pattern of products of 100-500 nucleotides in length (Shapiro et al., 25 Biochemistry 3727, 1986), but it has no significant ribonuclease activity in standard RNase assays, Id.

We have discovered that substances inhibiting at least the angiogenic activity of angiogenin can be used in methods and compositions for inhibiting tumor growth. Moreover, substances inhibiting the above described 18S, 28S rRNA-degrading enzymatic activity of angiogenin are effective tumor suppressants.

Accordingly, the invention features a therapeutic composition comprising a polypeptide comprising the amino acid sequence

Val-Asn-Pro-Ala-Leu-Ala-Glu-Leu-(Asn-Leu-Arg),

Ser-Asn-Glu-Leu-Gly-Asp-Val-Gly,

Trp-Leu-Trp-Leu-Ala-Asp-(Gln-Asp-Lys), or

(Trp or Val)-Leu-Trp-Leu-Ala-Asp-(Gln or Cys)-Asp-(Lys or Val),

wherein a segment of the polypeptide has angiogenin-inhibiting activity.

In a second aspect, the invention features engineered nucleic acid encoding a polypeptide comprising one of the above amino acid sequences. This polypeptide has a segment which has angiogenin-inhibited activity. Briefly, this aspect refers to any nucleic acid removed from its natural environment.

Other features and advantages of the invention will be apparent from the following description of the preferred embodiments thereof and from the claims.

The figures will first briefly be described:

Drawings

Fig. 1 is a graphical representation of the effect of angiogenin on inhibition of RNaseA activity toward uridylyl (3' , 5') adenosine (UpA) by PRI (Blackburn, 254 J Biol Chem 13484, 1979) as an example of an angiogenin inhibitor.

Fig. 2 is a graphical representation of elution of angiogenin (A) or angiogenin and PRI (B) from an HPLC column.

Fig. 3 shows the nucleotide sequence of PRI cDNA and the translated amino acid sequence.

Structure

Angiogenin

Angiogenin is a protein, having activities as described above, which can be obtained and purified as described by Fett et al., supra, and Shapiro et al., supra. Alternatively, angiogenin can be obtained and purified using recombinant DNA techniques. Kurachi et al., 24 Biochemistry 5494, 1985, describe the cDNA and gene encoding for angiogenin; this cDNA or gene can be expressed from a standard expression vector and the resulting angiogenin protein purified by standard procedure, e.g., in mammalian, yeast or various microbial expression systems. Id. at 5498.

Angiogenin Inhibitors

An angiogenin inhibitor is any compound able to inhibit the angiogenic activity of angiogenin. Preferably the inhibitor of angiogenin is a protein; most preferably it is a protein capable of binding to angiogenin and of inhibiting at least its biological angiogenic activity and preferably also its enzymatic 28S/18S rRNA-degrading activity. (The enzymatic activity is measured as described by Shapiro et al., 20pra. Briefly, 15-20$\mu$g RNA is incubated with about 1.9$\mu$M angiogenin at 37°C in either 30mM Hepes or 20mm Tris, pH 7.5, containing 30mM NaCl in a total volume of 13.5$\mu$l. The reaction is terminated after about 90 minutes using 49$\mu$l of a formamide/formaldehyde reagent, Id.) Preferably, the inhibitor is isolated from mammalian tissues, most preferably from human placental tissue.

One example of the inhibitor, PRI, can be isolated as described by Blackburn (254 J.Biol. Chem. 12,484, 1979). Proteins which are substantially equivalent to PRI, that is they have similar amino acid compositions, and have similar biological and enzymatic inhibitory activities, can be found not only in other human tissues but in other mammalian tissues. For example, the various inhibitors of RNAse A described by Burton et al., (19 Int. J. Peptide Protein Res. 372, 1982, herby incorporated by reference) may be suitable in this invention. These proteins have about 70 - 80% similarity in amino acid composition to PRI, as shown in Table 1 (taken from Burton et al., Id.) Such proteins are also angiogenin inhibitors for purposes of the invention. Methods for isolating and purifying these proteins are given below, using standard affinity chromatography methodology, or recombinant DNA techniques.

## Table 1

*Amino acid compositions of various liver RNase inhibitors and human placental RNase inhibitor*

| Amino acid | Beef | Mouse | Pig | Rat | Sheep | Human |
|---|---|---|---|---|---|---|
| | Liver | | | | | Placenta |
| Asx | 47 | 55 | 47 | 59 | 48 | 47 |
| Threonine | 21 | 22 | 25 | 23 | 19 | 16 |
| Serine | 45 | 43 | 43 | 43 | 42 | 45 |
| Glx | 62 | 63 | 64 | 60 | 62 | 64 |
| Proline | 15 | 16 | 15 | 17 | 17 | 17 |
| Glycine | 47 | 33 | 43 | 33 | 51 | 36 |
| Alanine | 37 | 30 | 35 | 29 | 35 | 34 |
| Valine | 24 | 20 | 19 | 22 | 23 | 24 |
| Methionine | 2 | 3 | 2 | 2 | 1–2 | 2–3 |
| Isoleucine | 9 | 13 | 9 | 10 | 10 | 12 |
| Leucine | 91 | 94 | 93 | 89 | 92 | 85 |
| Tyrosine | 5 | 5 | 4 | 5 | 4 | 4 |
| Phenylalanine | 4 | 3 | 2 | 5 | 4 | 6 |
| Histidine | 6 | 3 | 9 | 6 | 5 | 6 |
| Lysine | 14 | 21 | 15 | 20 | 15 | 17 |
| Arginine | 19 | 19 | 21 | 20 | 20 | 23 |
| 1/2 Cystine + cystine | 30 | 26–27 | 35 | 31 | 27 | 30 |
| Tryptophan | 5 | 5 | 5 | 5 | 6 | 5 |
| Total residues | 483 | 475 | 486 | 479 | 482 | 473 |

Alternatively, the gene encoding an inhibitor may be cloned by standard techniques. Such techniques include purifying the inhibitor, determining part of its amino acid sequence, creating a DNA probe capable of coding for this amino acid sequence, and using the probe to detect recombinant vectors in a cDNA or genomic library created from, e.g., placental cells. The cloned gene can then be expressed in any suitable expression vector in a suitable expression host cell, e.g., bacteria, yeast, or tissue culture cells. The recombinant inhibitor protein produced by those cells can then be purified from the culture supernatant or from the cells.

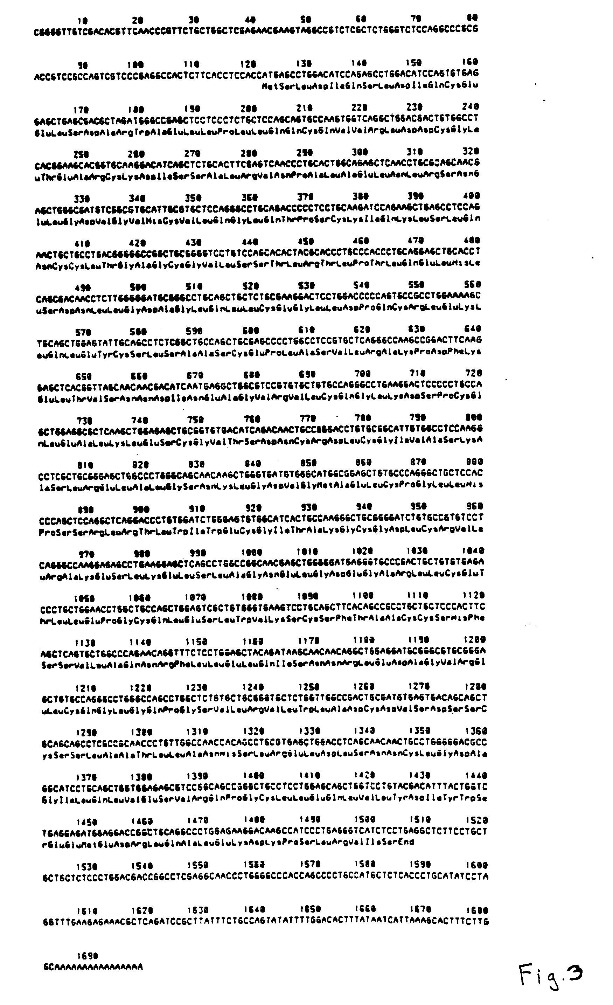

Suitable oligonucleotide probes for detecting clones expressing polypeptides according to the invention can be generated from the nucleic acid sequence given in Fig. 3. using, e.g., fragments of the Fig. 3 sequence (or the corresponding antisense sequence) that are at least 10, and preferably at least 16 bases in length. Probes with minor modifications (e.g. deviation at less than 3 of 16 positions) of the Fig. 3 sequence are acceptable. The following fragments are provided by way of example only; the corresponding antisense strands would be operative also:

5′-TGGCTGTGGCTGGCCGACCAGGAGAA-3′
5′-GTGCTCTGGTTGGCCGACTGCGAT-3′
5′-GTGAACCCTGCCCTGGCTGA-3′
5′-GTGAACCCTGGACTGGCAGA-3′

5′-AATGAGCTGGGCGATGTGGG-3′
5′-AACGAGCTGGGCGATGTCGG-3′

More generalized strands and corresponding antisense strands are:

```
5'-TGGBTNTGGBTNGCNGABCADGABAA-3'
5'-TTITCBTGITCCGCCAICCACTICCA-3'
           I  I      I


5'-AABGADBTNGGNGABGTNGG-3'
5'-CCCACITCCCCCAIBTCITT-3'
      I     I  I


5'-GTNAABCCNGCNBTNGCNGA-3'
5'-TCCGCCAICGCCGGITTCAC-3'
      I
```

In each of the above sequences B is C or T; I as inosine (which can base pair with A, T, or C) N is A, T, C or G, and

$$\frac{C}{I}$$

is C or I.

Inosine is used in the probe sequence in place of A, T, and C so that the number of probes to be synthesized as a mixture can be minimized without prejudicing their hybridizing ability to the natural gene or cDNA sequences.

One example of a method to isolate a cDNA clone of the gene encoding PRI is to use the above three antisense strand oligonucleotide probes under strigent hybridization conditions to probe a cDNA library of human placental DNA.

Another example of a procedure for isolating and screening cDNA clones expressing PRI is as follows. Antibodies are raised in rabbits against PRI purified as described above. The antibodies are purified by affinity chromatography, using PRI coupled to an activated solid matrix such as Sepharose (e.g. activated by CNBr).

Any clones which hybridize to one or more of the probes, and preferably hybridize to all three, or any clones that express proteins that bind to anti-PRI antibodies, are potential clones of the PRI-encoding gene. To determine whether the clones do encode PRI, they can be purified, followed by re-checking of re-hybridization with the probes or of expression of proteins that bind anti-PRI antibodies.

Ultimately, the clones can be sequenced and the sequences compared to see if they correlate with the known properties of PRI, i.e., the amino acid sequence, molecular weight and amino acid composition. Expression of PRI can then be achieved by insertion of the cDNA clone into an expression vector and the recombinant PRI which is expressed, isolated, and purified. All such clones contain engineered DNA, that is, DNA taken from its natural environment and inserted into a vector, such as a plasmid or phase, or even within the genome of an organism. Thus, the engineered DNA is no longer surrounded by naturally occuring sequences on either side of it. Generally such engineered DNA is constructed using recombinant DNA techniques, and does not include naturally occurring DNA in which, for example, a translocation of chromosomal DNA in vivo has changed the environment of DNA; however it does include such natural events which occur after the DNA has been manipulated in some way by recombinant DNA methodology.

We have determined the nucleotide sequence of human PRI cDNA and the amino acid sequence of human PRI inferred from that nucleotide sequence. We have confirmed a portion of that amino acid sequence by Edman degradation of PRI tryptic peptides.

Fig 3 shows the PRI cDNA sequence and translated amino acid sequence. A bacterium, E. coli DH5α, transformed with vector pUC18-PRI(A) containing the cDNA of Fig. 3 has been deposited with the American Type Culture Collection in Maryland under access number 67668 on March 31, 1988.

Angiogenin inhibitors can be obtained using the PRI information in Fig. 3, e.g., by producing recombinant PRI inhibitor using standard techniques for culturing cells that include the PRI cDNA on a suitable expression vector, and purifying the PRI from the culture supernatant.

Fragments and variants of the PRI of Fig. 3 sequence are also suitable. For example, PRI can be fragmented (e.g. by tryptic digestion) and the resulting fragments (purified by HPLC) can be assayed as described elsewhere in this application for angiogen inhibitory activity. Specific PRI fragments of use are as follows:

Val-Asn-Pro-Ala-Leu-Ala-Glu-Leu-Asn-Leu-Arg;

Ser-Asn-Glu-Leu-Gly-Asp-Val-Gly;

(Trp or Val)-Leu-Trp-Leu-Ala-Asp-(Cys or Gln)-Asp-(Val or Lys).

PRI from other mammals, such as the ones shown in Table 1, can be obtained and analyzed by cloning them from other mammals and providing angiogenic fragments as described above.

Once cloned, the naturally occurring gene may also be modified by standard techniques, for example, by altering the amino acid sequence of the inhibitor, so long as the inhibitor retains the ability to inhibit the biological angiogenic activity of angiogenin. Such alterations may be designed to increase the binding ability of the inhibitor for angiogenin. Further, related inhibitor-encoding genes can be isolated by using a part of the above described cloned gene encoding an inhibitor as a probe for libraries, constructed by standard techniques, of other animal genomes.

In order to isolate other inhibitors, one possible procedure includes detecting proteins able to bind angiogenin, for example, by binding angiogenin to a column and passing a sample containing potential inhibitor proteins through this column. (RNaseA can be used in place of angiogenin, as described by Blackburn 254 J. Biol. Chem. 12488, 1979.) Bound protein can then be eluted, for example, by using 0.1M sodium acetate pH 5.0 containing 3M NaCl, 15% glycerol, 1mM EDTA and 5mM DTT, which is able to dissociate the angiogenin/inhibitor complex. The eluted protein can then be purified and tested, as described below, to see if the released proteins inhibit the angiogenic activity of angiogenin, or altenatively if they inhibit its enzymatic activity. The active protein(s) may then be purified by standard procedure.

Further, it is possible to use segments of inhibitors, such as segments of PRI, which retain the biological inhibiting character of the native inhibitor. Such segments can be created by standard procedure using proteases to digest natural inhibitors to produce active segments; or by using recombinant DNA techniques to remove non essential parts of the structural gene or cDNA encoding the inhibitor, and then expressing this engineered DNA to produce a modified inhibitor.

Inhibitory Activity

Below is an example of in vitro measurements relative to the inhibitory effect of PRI on angiogenin. This example is not meant to be limiting for the invention and those skilled in the art will realize that these examples are indicative of whether other inhibitors or segments thereof can be used in the methods described below.

Measurement of Inhibition of the Enzymatic Activity of Angiogenin

Angiogenin and PRI were purified generally as described by Fett et al. and Shapiro et al., cited above. The enzymatic activity of angiogenin was measured as described by Shapiro et al., supra. Briefly, angiogenin catalyzes the cleavage of 28S and 18S rRNA to form products of 100 to 500 nucleotides in length. These products are stable and include a large, approximately 500 bases, segment which is visible in an agarose gel. In one experiment, 12 μg of RNA was incubated with or without angiogenin and/or PRI at 37°C in 33 mM Hepes, and 33 mM NaCl, pH7.5. After 30 min the reaction was terminated, as described by Shapiro et al., supra, the samples run on a 1.1% agarose gel, and stained with ethidium bromide. 0.96 μM PRI completely inhibited the enzymatic degradation of rRNA by 0.8 μM angiogenin. PRI alone had no activity on rRNA. Thus, PRI is a potent inhibitor of the enzymatic activity of angiogenin being active at only just over a 1:1 ratio with angiogenin.

Stability of the Angiogenin/PRI Complex

For therapeutic use of the angiogenin inhibitor it is advantageous to use an inhibitor that binds strongly to angiogenin and reduces its biological activity. PRI is such an inhibitor.

RNaseA also binds to PRI and competes with angiogenin for binding PRI in solution. The enzymatic activity of RNaseA is also inhibited by PRI. These properties can be used to determine the stability of the PRI/angiogenin complex, as follows.

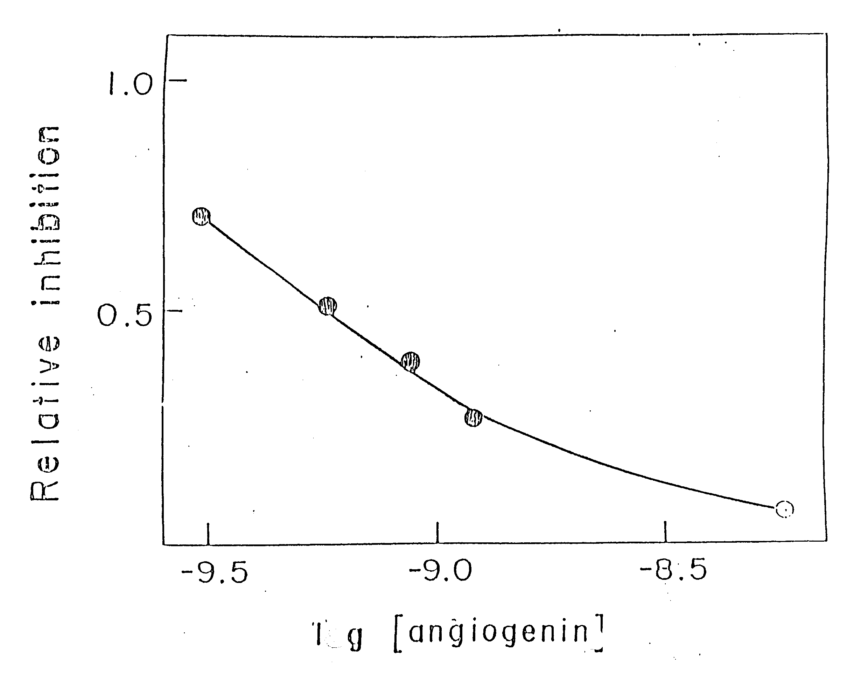

UpA is an excellent substrate for RNaseA but not for angiogenin. In one experiment, a varying amount of angiogenin was added to a mixture of 0.27 nM RNaseA and 0.20 nM PRI, with the RNaseA and angiogenin being mixed together first, followed by PRI for 5 min. at 25°C, and then the UpA substrate (0.2mM, in a buffer of 10 $\mu$g/ml human serum albumin (HSA), 0.1M 2-(N-morpholino)-ethane-sulfonic acid, 0.1M NaCl, and 1mM EDTA pH6.0). The more angiogenin present in the mixture the more PRI that will be bound to it, and thus the less PRI that will be bound to RNaseA and able to inhibit its enzymatic activity. Further, the stronger the binding of PRI to angiogenin than to RNaseA, the greater the reduction in inhibition of RNaseA activity, since PRI will then preferentially bind to angiogenin. The results are shown in Fig. 1. Relative inhibition is calculated as (Vo-Va)/(Vo-Vr) where Vo denotes velocity of RNaseA activity in the absence of PRI, Vr denotes velocity in the presence of PRI with no angiogenin added, and Va is velocity in the presence of PRI with angiogenin added. At 5.8nM angiogenin there is essentially no inhibition of RNaseA activity. Thus, a large excess of angiogenin is needed to remove all the PRI from reacting with and inhibiting the RNaseA.

In a related experiment, the $K_i$ for angiogenin and PRI can be estimated using the above method and modifying it so that the PRI and angiogenin are firstly preincubated for 10 minutes, followed by addition of RNaseA and UpA. This assay determines the level of free inhibitor remaining after incubation of PRI and angiogenin. Thus, if the dissociation of PRI and angiogenin is slow there will be little inhibition of RNaseA activity. At a ratio of angiogenin:PRI of 1:1.2 no free PRI was detected. Thus, PRI and angiogenin are tightly bound and appear to have a $K_i$ less than 0.1nM.

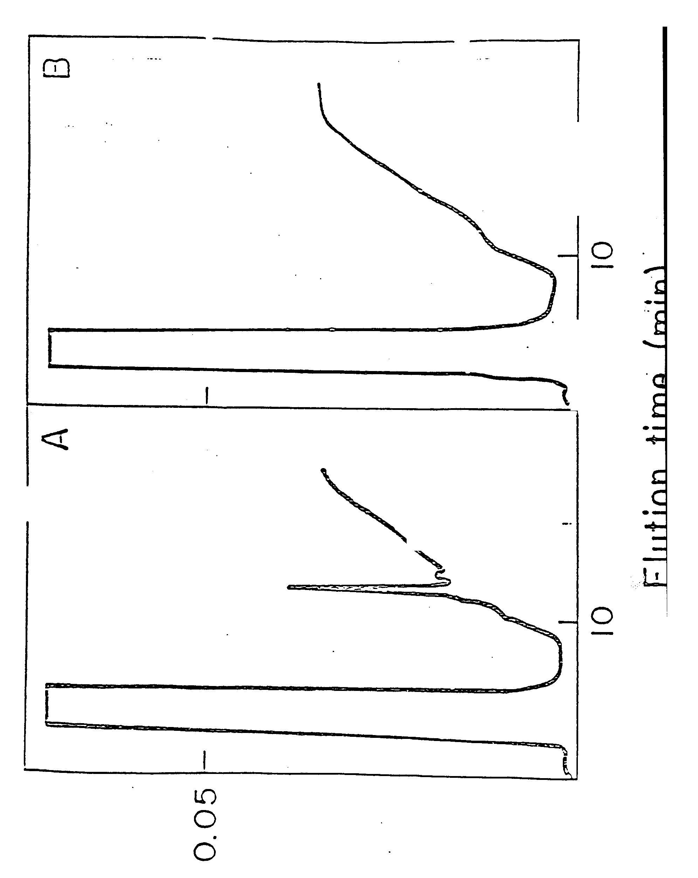

The strength of binding of PRI and angiogenin was also demonstrated by cation-exchange HPLC. This process can distinguish free angiogenin from angiogenin/PRI complex. A Synchropak CM 300 column (250 x 4.1 mm; Sychrom, Inc.), a Waters Associates liquid chromatography system and a Hewlett-Packard 3390A integrator were used; the results are shown in Fig. 2. Samples of 1ml in 0.1M Tris, pH7, containing 1mM EDTA and 10 $\mu$g HSA were eluted with a 10 min. linear gradient from 220-620 mM NaCl in 20 mM sodium phosphate, pH7, at a flow rate of 1ml/min.; effluents were monitored at 214 nm. In panel A is shown elution of 0.64 $\mu$g angiogenin; in panel B the elution of 0.64 $\mu$g angiogenin and 12 $\mu$g PRI. There is no detectable free angiogenin shown in panel B. Addition of RNaseA to the angiogenin - PRI mixture at 32-fold excess did not produce free angiogenin, even after 17 h incubation. Thus, dissociation of angiogenin/PRI appears to have a half life of greater than 1 day.

Inhibition of Angiogenic Activity

Angiogenesis (i.e., angiogenic activity) was assessed by a modification of the chick embryo chorioallantoic membrane (CAM) assay of Knighton et al. 35 Brit. J.Cancer 347 (1977) described by Fett et al., supra. For this assay PRI was desalted using an Amicon Centricon-10 microconcentrator to dilute the buffer at least 500 fold. This removes interfering components, from the PRI solution, which may adversely affect the experiment or may harm the egg itself. The results of adding PRI to the angiogenin are shown in Table 2 below.

Data for experiments #1 and #2 represent composites of results obtained in 2 and 3 sets of assays, respectively. Between 10 and 20 eggs were utilized for each of the three experimental groups within each set. The following amounts of angiogenin and PRI were employed: Experiment #1, 75 ng and 2 $\mu$g, respectively; Experiment #2, 46 ng and 700 ng; and Experiment #3, 25 ng and 180 ng.

## Table 2

### % Positives (Total Number of Eggs)

| Sample | Experiment | #1 | #2 | #3 |
|---|---|---|---|---|
| Angiogenin | | 58 (24) | 52 (54) | 62 (13) |
| PRI | | 17 (29) | 33 (48) | 17 (12) |
| Angiogenin + PRI | | 15 (26) | 25 (52) | 7 (14) |

From these results it is evident that excess PRI decreases angiogenin activity to a level indistinguishable from that observed with the buffer and inhibitor alone controls.

Inhibition of Tumor Growth

For immunotherapeutic studies, male nude (nu/nu) mice (Charles River Laboratories) were maintained under laminar flow conditions, and were age- and cage-matched (5 animals per cage) prior to experimentation. Experimental animals (5-10 per group) were injected subcutaneously (S.C.) with 5 x $10^5$ HT-29 human colon adenocarcinoma cells (Fogh et al., In Human Tumor Cells In Vitro, pp115-160 ed. Fogh, Plenum Press, New York., 1975) on day 0. Also on day 0, animals were treated with either buffer control (the buffer used to store PRI dialysed against phosphate buffered saline; 0.2g/l KCl, 0.2g/l $KH_2PO_4$, 8g/l NaCl and 2.16g/l $Na_2HPO_4.7H_2O$, pH7.4 at 37°C, PBSA 100 $\mu$l) or placental inhibitor (100 $\mu$l) at varying dosages by S.C. or intraperitoneal (I.P.) injection. Treatment regimens included daily injections (10-11 doses) or injections at 2-3 day intervals (5-10 doses) depending on the experimental protocol. Assessment of animal health, weight, tumor size as well as photographic records were recorded 2-3 times per week. At the termination of the experiments blood and tissue samples were collected for immunological and histological evaluation.

In one experiment, forty male nu/nu mice were injected S.C. with 5 x $10^5$ HT-29 cells on day 0. Additionally, on day 0 mice 1-10 (Group 1) received 100 $\mu$l of control buffer; mice 11-20 (Group 2) received 10 $\mu$g in 100 $\mu$l of placental inhibitor; mice 21-30 (Group 3) received 1.0 $\mu$g in 100 $\mu$l of placental inhibitor; and mice 31-40 received 0.1 $\mu$g in 100 $\mu$l of placental inhibitor. Groups 1 and 2 received further injections of buffer or 10 $\mu$g of placental inhibitor, respectively, on days 1, 4, 6, 8, 18, 20, and 22. On days 1, 4, 6 and 8, Groups 3 and 4 received placental inhibitor at dosages of 1.0 $\mu$g or 0.1 $\mu$g, respectively. All treatment injections were given I.P. After 60 days, only animals 13, 14, 17, 18, 20 and 24 remained tumor free. Thus, inoculation with 10 $\mu$g PRI prevented tumor formation in about 60% of animals.

Inhibition of both the biological and enzymatic activities of angiogenin by an inhibitor has important mechanistic, physiologic and pharmacologic implications. It is consistent with the hypothesis that these two actions of angiogenin are interrelated, as suggested previously by the simultaneous loss of both activities upon carboxymethylation by bromoacetate at pH 5.5. Shapiro et al., supra. Further it raises the possibility that such inhibitors may play a role in the in vivo regulation of angiogenin. The angiogenin/PRI interaction likely involves regions of angiogenin separated widely in the three-dimensional structure, many of them outside the active center. Thus, conservation of residues necessary for enzymatic activity alone probably cannot account for the strength of the interaction.

This implies that the capacity of angiogenin to bind PRI has been maintained independently during evolution. Apparently, binding by PRI and other inhibitors reflects a physiologically relevant control mechanism having pharmacologic and therapeutic potential. For example, as shown above, these inhibitors are active in prevention of tumor formation in mice. Apparently they are suitable for injection into humans or other animals in order to prevent tumor growth, and other diseases associated with neovascularization, e.g., diabetic retinopathy, rheumatoid arthritis, and Kaposi's sarcoma. That is, they are suitable for treating disorders where vascularization plays an important role in the pathophysiology of diseases such as solid tumors, hemangiomata and psoriasis.

Preferably, they are injected intravascularly, most preferably intravenously, or injected intraperitoneally in a physiologically compatible medium (such as PBSA, or those buffers and agents described by Krakoft, Ca-A Cancer Journal for Clinicians, March/April 1987, Vol. 37:93), suitable for administration to an animal in a sufficient quantity, e.g., about 10-10,000 $\mu$g/kg body weight of the animal, to inhibit naturally occuring biological activity of angiogenin within the animal. Alternatively, they can be administered topically to a specific area, or injected subcutaneously.

We have found that these inhibitors are stable for at least 2 weeks even at concentrations as low as 8$\mu$M when they are in a purified form; there is no need to include DTT in the storage buffer or the therapeutic composition being administered, however, natural agents, which are physiologically compatible, e.g., N-acetyl cysteine or cysteine or cysteamine may be used. For administration it is important that the buffer fluid not injure the patient. It is possible that these inhibitors may be used prophylatically at a low concentration. Thus, for example, it is appropriate to administer 10-10,000 $\mu$g/kg body weight to patients at risk of cancer development, e.g., patients who have had a primary tumor removed.

**Claims**

1. A therapeutic composition comprising a polypeptide comprising the amino acid sequence
   Val-Asn-Pro-Ala-Leu-Ala-Glu-Leu-(Asn-Leu-Arg);
   Ser-Asn-Glu-Leu-Gly-Asp-Val-Gly; or
   Trp-Leu-Trp-Leu-Ala-Asp-(Gln-Asp-Lys), wherein a segment of said polypeptide has angiogenin inhibiting activity.

2. A therapeutic composition comprising a polypeptide comprising the amino acid sequence
   Val-Asn-Pro-Ala-Leu-Ala-Glu-Leu-(Asn-Leu-Arg);
   Ser-Asn-Glu-Leu-Gly-Asp-Val-Gly;
   (Trp or Val)-Leu-Trp-Leu-Ala-Asp-(Cys or Gln)-Asp-(Val or Lys), wherein a segment of said polypeptide has angiogenin inhibiting activity.

3. Engineered nucleic acid encoding a polypeptide comprising the amino acid sequence
   Val-Asn-Pro-Ala-Leu-Ala-Glu-Leu-(Asn-Leu-Arg);
   Ser-Asn-Glu-Leu-Gly-Asp-Val-Gly; or
   Trp-Leu-Trp-Leu-Ala-Asp-(Gln-Asp-Lys), wherein a segment of said polypeptide has angiogenin inhibiting activity.

4. Engineered nucleic acid encoding a polypeptide comprising the amino acid sequence
   Val-Asn-Pro-Ala-Leu-Ala-Glu-Leu-(Asn-Leu-Arg);
   Ser-Asn-Glu-Leu-Gly-Asp-Val-Gly;
   (Trp or Val)-Leu-Trp-Leu-Ala-Asp-(Cys or Gln)-Asp-(Val or Lys), wherein a segment of said polypeptide has angiogenin inhibiting activity.

**Patentansprüche**

1. Therapeutische Zusammensetzung, umfassend ein Polypeptid, umfassend die Aminosäuresequenz:
   Val-Asn-Pro-Ala-Leu-Ala-Glu-Leu-(Asn-Leu-Arg),
   Ser-Asn-Glu-Leu-Gly-Asp-Val-Gly oder
   Trp-Leu-Trp-Leu-Ala-Asp-(Gln-Asp-Lys),
   wobei ein Segment des genannten Polypeptids Angiogenin-inhibierende Aktivität hat.

2. Therapeutische Zusammensetzung, umfassend ein Polypeptid, umfassend die Aminosäuresequenz:
   Val-Asn-Pro-Ala-Leu-Ala-Glu-Leu-(Asn-Leu-Arg),
   Ser-Asn-Glu-Leu-Gly-Asp-Val-Gly,
   (Trp oder Val)-Leu-Trp-Leu-Ala-Asp-(Cys oder Gln)-Asp-(Val oder Lys),
   wobei ein Segment des genannten Polypeptids Angiogenin-inhibierende Aktivität hat.

3. Technisch hergestellte Nucleinsäure, die für ein Polypeptid codiert, das die Aminosäuresequenz
   Val-Asn-Pro-Ala-Leu-Ala-Glu-Leu-(Asn-Leu-Arg),
   Ser-Asn-Glu-Leu-Gly-Asp-Val-Gly oder
   Trp-Leu-Trp-Leu-Ala-Asp-(Gln-Asp-Lys) umfaßt,
   wobei ein Segment des genannten Polypeptids Angiogenin-inhibierende Aktivität hat.

4. Technisch hergestellte Nucleinsäure, die für ein Polypeptid codiert, das die Aminosäuresequenz
   Val-Asn-Pro-Ala-Leu-Ala-Glu-Leu-(Asn-Leu-Arg),
   Ser-Asn-Glu-Leu-Gly-Asp-Val-Gly,
   (Trp oder Val)-Leu-Trp-Leu-Ala-Asp-(Cys oder Gln)-Asp-(Val oder Lys) umfaßt,
   wobei ein Segment des genannten Polypeptids Angiogenin-inhibierende Aktivität hat.

**Revendications**

1. Composition thérapeutique comprenant un polypeptide comprenant l'une des séquences d'amino-acides suivantes :
   Val-Asn-Pro-Ala-Leu-Ala-Glu-Leu-(Asn-Leu-Arg);
   Ser-Asn-Glu-Leu-Gly-Asp-Val-Gly; ou

Trp-Leu-Trp-Leu-Ala-Asp-(Gln-Asp-Lys), composition dans laquelle un segment dudit polypeptide présente une activité inhibitrice vis-à-vis de l'angiogénine.

2. Composition thérapeutique comprenant un polypeptide comprenant l'une des séquences d'aminoacides suivantes :
Val-Asn-Pro-Ala-Leu-Ala-Glu-Leu-(Asn-Leu-Arg);
Ser-Asn-Glu-Leu-Gly-Asp-Val-Gly;
(Trp ou Val)-Leu-Trp-Leu-Ala-Asp-(Cys ou Gln)-Asp-(Val ou Lys), composition dans laquelle un segment dudit polypeptide présente une activité inhibitrice vis-à-vis de l'angiogénine.

3. Acide nucléique recombinant codant pour un polypeptide comprenant l'une des séquences d'aminoacides suivantes :
Val-Asn-Pro-Ala-Leu-Ala-Glu-Leu-(Asn-Leu-Arg);
Ser-Asn-Glu-Leu-Gly-Asp-Val-Gly; ou
Trp-Leu-Trp-Leu-Ala-Asp-(Gln-Asp-Lys), acide nucléique dans lequel un segment dudit polypeptide présente une activité inhibitrice vis-à-vis de l'angiogénine.

4. Acide nucléique recombinant codant pour un polypeptide comprenant l'une des séquences d'amino-acides suivantes :
Val-Asn-Pro-Ala-Leu-Ala-Glu-Leu-(Asn-Leu-Arg);
Ser-Asn-Glu-Leu-Gly-Asp-Val-Gly;
(Trp ou Val)-Leu-Trp-Leu-Ala-Asp-(Cys ou Gln)-Asp-(Val ou Lys), acide nucléique dans lequel un segment dudit polypeptide présente une activité inhibitrice vis-à-vis de l'angiogénine.

```
          10        20        30        40        50        60        70        80
CGGGTTGTCGACACGTTCAACCCGTTCTGCTGGCTCGAGAACGAAGTAGGCCGTCTCGCTCTGGGTCTCCAGGCCCGCG

          90       100       110       120       130       140       150       160
ACCGTCCGCCAGTCGTCCCGAGGCCACTCTTCACCTCCACCATGAGCCTGGACATCCAGAGCCTGGACATCCAGTGTGAG
                                                MetSerLeuAspIleGlnSerLeuAspIleGlnCysGlu

         170       180       190       200       210       220       230       240
GAGCTGAGCGACGCTAGATGGGCCGAGCTCCTCCCTCTGCTCCAGCAGTGCCAAGTGGTCAGGCTGGACGACTGTGGCCT
GluLeuSerAspAlaArgTrpAlaGluLeuLeuProLeuLeuGlnGlnCysGlnValValArgLeuAspAspCysGlyLe

         250       260       270       280       290       300       310       320
CACGGAAGCACGGTGCAAGGACATCAGCTCTGCACTTCGAGTCAACCCTGCACTGGCAGAGCTCAACCTGCCGCAGCAACG
uThrGluAlaArgCysLysAspIleSerSerAlaLeuArgValAsnProAlaLeuAlaGluLeuAsnLeuArgSerAsnG

         330       340       350       360       370       380       390       400
AGCTGGGCGATGTCGGCGTGCATTGCGTGCTCCAGGGCCTGCAGACCCCCTCCTGCAAGATCCAGAAGCTGAGCCTCCAG
luLeuGlyAspValGlyValHisCysValLeuGlnGlyLeuGlnThrProSerCysLysIleGlnLysLeuSerLeuGln

         410       420       430       440       450       460       470       480
AACTGCTGCCTGACGGGGGCCGGCTGCGGGGTCCTGTCCAGCACACTACGCACCCTGCCCACCCTGCAGGAGCTGCACCT
AsnCysCysLeuThrGlyAlaGlyCysGlyValLeuSerSerThrLeuArgThrLeuProThrLeuGlnGluLeuHisLe

         490       500       510       520       530       540       550       560
CAGCGACAACCTCTTGGGGGATGCGGGCCTGCAGCTGCTCTGCGAAGGACTCCTGGACCCCCAGTGCCGGCCTGGAAAAGC
uSerAspAsnLeuLeuGlyAspAlaGlyLeuGlnLeuLeuCysGluGlyLeuLeuAspProGlnCysArgLeuGluLysL

         570       580       590       600       610       620       630       640
TGCAGCTGGAGTATTGCAGCCTCTCGGCTGCCAGCTGCGAGCCCCTGGCCTCCGTGCTCAGGGCCAAGCCGGACTTCAAG
euGlnLeuGluTyrCysSerLeuSerAlaAlaSerCysGluProLeuAlaSerValLeuArgAlaLysProAspPheLys

         650       660       670       680       690       700       710       720
GAGCTCACGGTTAGCAACAACGACATCAATGAGGCTGGCGTGCGTGTGCTGTGCCAGGGCCTGAAGGACTCCCCCTGCCA
GluLeuThrValSerAsnAsnAspIleAsnGluAlaGlyValArgValLeuCysGlnGlyLeuLysAspSerProCysGl

         730       740       750       760       770       780       790       800
GCTGGAGGCCCTCAAGCTGGAGAGCTGCGGTGTGACATCAGACAACTGCCGGGACCTGTGCGGCATTGTGGCCTCCAAGG
nLeuGluAlaLeuLysLeuGluSerCysGlyValThrSerAspAsnCysArgAspLeuCysGlyIleValAlaSerLysA

         810       820       830       840       850       860       870       880
CCTCGCTGCGGGAGCTGGCCCTGGGCAGCAACAAGCTGGGTGATGTGGGCATGGCGGAGCTGTGCCCAGGGCTGCTCCAC
laSerLeuArgGluLeuAlaLeuGlySerAsnLysLeuGlyAspValGlyMetAlaGluLeuCysProGlyLeuLeuHis

         890       900       910       920       930       940       950       960
CCCAGCTCCAGGCTCAGGACCCTGTGGATCTGGAGTGTGCCATCACTGCCAAGGGCTGCGGGGATCTGTGCCGTGTCCT
ProSerSerArgLeuArgThrLeuTrpIleTrpGluCysGlyIleThrAlaLysGlyCysGlyAspLeuCysArgValLe

         970       980       990      1000      1010      1020      1030      1040
CAGGGCCAAGGAGAGCCTGAAGGAGCTCAGCCTGGCCGGCAACGAGCTGGGGGATGAGGGTGCCCGACTGCTGTGTGAGA
uArgAlaLysGluSerLeuLysGluLeuSerLeuAlaGlyAsnGluLeuGlyAspGluGlyAlaArgLeuLeuCysGluT

        1050      1060      1070      1080      1090      1100      1110      1120
CCCCTGCTGGAACCTGCTGCCAGCTGGAGTCGCTGTGGGTGAAGTCCTGCAGCTTCACAGCCGCCTGCTGCTCCCACTTC
hrLeuLeuGluProGlyCysGlnLeuGluSerLeuTrpValLysSerCysSerPheThrAlaAlaCysCysSerHisPhe

        1130      1140      1150      1160      1170      1180      1190      1200
AGCTCAGTGCTGGCCCAGAACAGGTTTCTCCTGGAGCTACAGATAAGCAACAACAGGCTGGAGGATGCGGGCGTGCGGGA
SerSerValLeuAlaGlnAsnArgPheLeuLeuGluLeuGlnIleSerAsnAsnArgLeuGluAspAlaGlyValArgGl

        1210      1220      1230      1240      1250      1260      1270      1280
GCTGTGCCAGGGCCTGGGCCAGCCTGGCTCTGTGCTGCGGGTGCTCTGGTTGGCCGACTGCGATGTGAGTGACAGCAGCT
uLeuCysGlnGlyLeuGlyGlnProGlySerValLeuArgValLeuTrpLeuAlaAspCysAspValSerAspSerSerC

        1290      1300      1310      1320      1330      1340      1350      1360
GCAGCAGCCTCGCCGCCAACCCTGTTGGCCAACCACAGCCTGCGTGAGCTGGACCTCAGCAACAACTGCCTGGGGGACGCC
ysSerSerLeuAlaAlaThrLeuLeuAlaAsnHisSerLeuArgGluLeuAspLeuSerAsnAsnCysLeuGlyAspAla

        1370      1380      1390      1400      1410      1420      1430      1440
GGCATCCTGCAGCTGGTGGAGAGCGTCCGGGCAGCCGGGCTGCCTCCTGGAGCAGCTGGTCCTGTACGACATTTACTGGTC
GlyIleLeuGlnLeuValGluSerValArgGlnProGlyCysLeuLeuGluGlnLeuValLeuTyrAspIleTyrTrpSe

        1450      1460      1470      1480      1490      1500      1510      1520
TGAGGAGATGGAGGACCGGCTGCAGGCCCTGGAGAAGGACAAGCCATCCCTGAGGGTCATCTCCTGAGGCTCTTCCTGCT
rGluGluMetGluAspArgLeuGlnAlaLeuGluLysAspLysProSerLeuArgValIleSerEnd

        1530      1540      1550      1560      1570      1580      1590      1600
GCTGCTCTCCCTGGACGACCGGCCTCGAGGCAACCCTGGGGCCCACCAGCCCCTGCCATGCTCTCACCCTGCATATCCTA

        1610      1620      1630      1640      1650      1660      1670      1680
GGTTTGAAGAGAAACGCTCAGATCCGCTTATTTCTGCCAGTATATTTTGGACACTTTATAATCATTAAAGCACTTTCTTG

        1690
GCAAAAAAAAAAAAAAAA
```

Fig.3

13